# EUROPÄISCHE PATENTSCHRIFT

(11) **EP 3 166 614 B1**
(45) Veröffentlichungstag und Bekanntmachung des Hinweises auf die Patenterteilung: **16.06.2021**
(21) Anmeldenummer: 15727870.6
(22) Anmeldetag: 15.04.2015
(51) Int. Cl.: A61K 31/568, A61K 31/4196, A61K 31/565, A61K 31/5685, A61K 31/57, A61P 35/00

(54) **ZUSAMMENSETZUNG ZUR PRÄVENTION UND THERAPIE VON TUMORERKRANKUNGEN**
COMPOSITION FOR PREVENTING AND TREATING OF TUMORS
COMPOSITIONS POUR TRAITER DES TUMEURS

(30) Priorität: 15.04.2014 DE 102014005513
(43) Veröffentlichungstag der Anmeldung: 17.05.2017
(73) Patentinhaber: Sanoxsys GmbH, 80799 München (DE)
(72) Erfinder: SCHALLER, Gerhard, 80799 München (DE)
(74) Vertreter: Baumbach, Friedrich
(86) Internationale Anmeldenummer: PCT/DE2015/000182
(87) Internationale Veröffentlichungsnummer: WO 2015/158321

(56) Entgegenhaltungen:
- WO-A1-99/59595
- WO-A1-03/053438
- WO-A1-2007/128561
- WO-A2-2009/093262
- FR-A1- 2 964 323
- US-A1- 2005 180 923
- BOULOUX ET AL: "Testim(R) 1% testosterone gel for the treatment of male hypogonadism", CLINICAL THERAPEUTICS, EXCERPTA MEDICA, PRINCETON, NJ, US, Bd. 27, Nr. 3, 1. März 2005 (2005-03-01), Seiten 286-298, XP027641072, ISSN: 0149-2918 [gefunden am 2005-03-01]

## Beschreibung

Die Erfindung betrifft ein Mittel zur Prävention und Therapie hormonsensibler Tumorgewebe, insbesondere zur Verhinderung bzw. der Hemmung von Mammakarzinomen oder Prostatakarzinomen. Anwendungsgebiete der Erfindung sind die pharmazeutische Industrie und die Lebenswissenschaften, insbesondere die Medizin und die Medizintechnologie.

### Stand der Technik

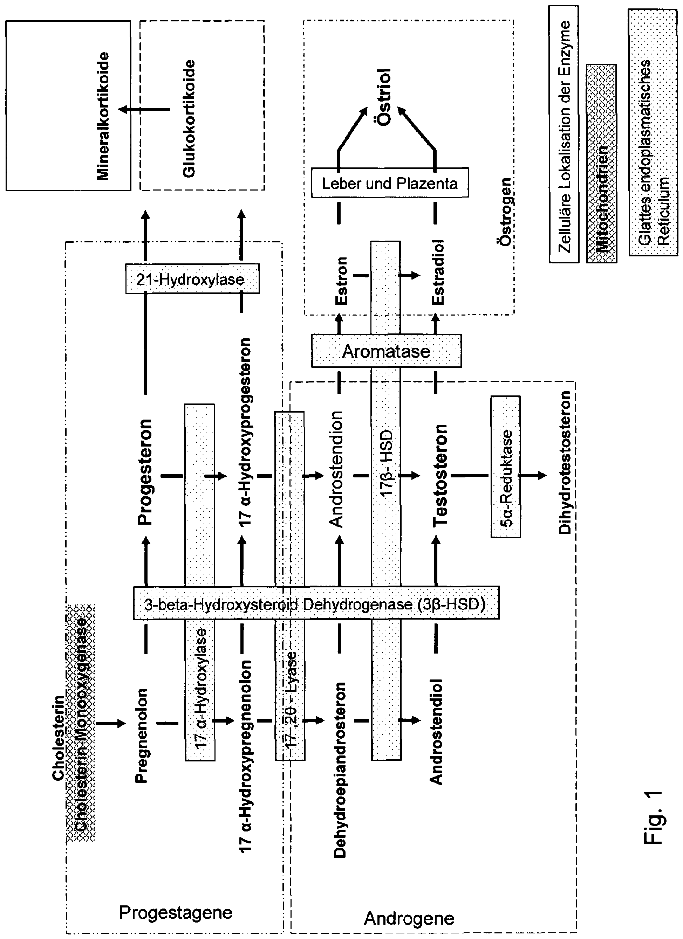

Bei der Behandlung einer Vielzahl von Karzinomen spielt oftmals der medikamentöse Eingriff in den Hormonhaushalt eine bedeutende Rolle. Herausragendes Beispiel hierfür ist das Mammakarzinom. Genau genommen handelt es sich dabei immer um eine antihormonelle Therapie. Die Interaktion der beiden proliferationssteigernden Steroidhormone Estradiol (E2) oder dem Estron (E1) mit den Estrogenrezeptoren wird behindert. Dies kann auf zwei verschiedenen Weisen geschehen. Mit Hilfe der selektiven Estrogenrezeptormodulatoren (SERM), wie zum Beispiel dem Tamoxifen, wird das Estrogen kompetetiv vom Rezeptor verdrängt oder die Synthese der Estrogene wird durch die Hemmung der Aromatase nahezu vollständig zum Erliegen gebracht. Als Übersicht ist in Figur 1 der Steroidstoffwechsel schematisch dargestellt.

Die Gabe von Hormonen zur Behandlung von Karzinomen hat dagegen nahezu keine Bedeutung. Gleichwohl lassen sich *in vitro* und *in vivo* mit Hilfe verschiedener Steroidhormone nicht nur die Proliferation (Krebswachstum) anregen sondern diese auch hemmen.

Folgende Präparate bzw. Hormone spielen im Organismus bei einer Tumorbehandlung eine wichtige Rolle.

### Aromatasehemmer

Die Aromatase ist ein Enzym, das den entscheidenden Schritt der Umwandlung der beiden Androgene Testosteron und Androstendion in Estradiol (E2) und Estron (E1) katalysiert (Figur 1). Zur Behandlung des Estrogenrezeptor exprimierenden Mammakarzinoms wurden die Aromatasehemmer Exemestan, Anastrozol und Letrozol zugelassen. Ihre Gabe verhindert in der Postmenopause die Bildung von Östrogenen aus den androgenen Vorstufen der Nebenniere oder des Fettgewebes. Die Aromatasehemmer führen durch den massiven Estrogenentzug (E2 und E1) zu einer Hemmung des Brustkrebswachstums (Howell et al, 2005)

### Estriol (E3)

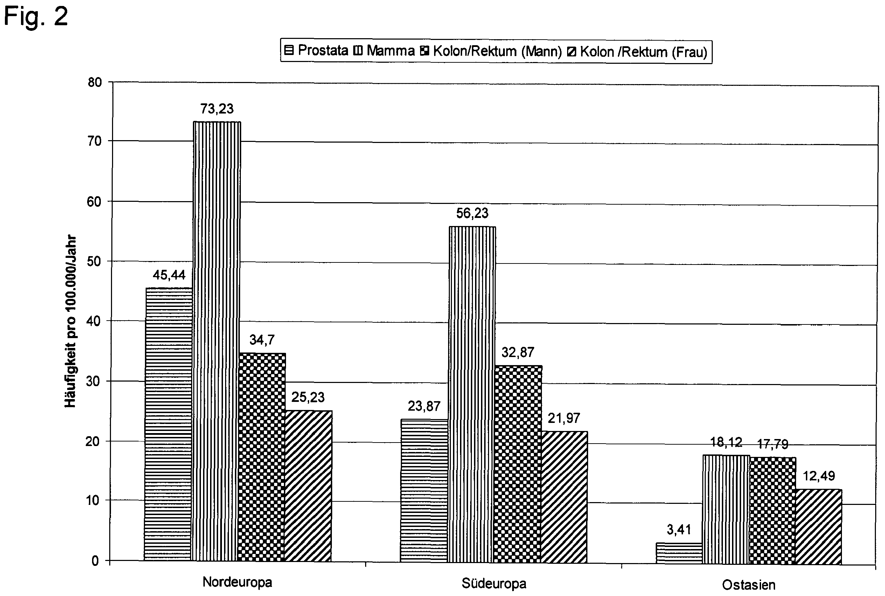

Der Estrogenrezeptor-β ist ein potenter Tumorsuppressor und spielt eine entscheidende Rolle bei vielen Krebsarten, wie zum Beispiel dem Prostatakarzinom (Stettner et al., 2007). Der ER-β wird prädominat durch das Estriol (Zhu et al., 2006), aber auch durch Pytoestrogene wie Genistein oder Mariendistel aktiviert. Die Japanerinnen aktivieren zeitlebens den ER-β, da sie täglich das Genistein in Form von fermentiertem Soja in ihrer Nahrung aufnehmen. Dementsprechend haben die Japanerinnen die niedrigsten Brustkrebsraten der Welt, wie in gleicher Weise die Männer die niedrigsten Prostatakarzinomraten haben (Wu et al., 2002) (Figur 2). Das Estriol entsteht in nennenswerten Mengen nur in der Schwangerschaft. Zur Synthese bedarf es der estrogenen Vorstufen aus der kindlichen Leber und der Syntheseleistung der Plazenta. Außerhalb der Schwangerschaft lassen sich nur Spuren des nicht konjugierten Hormons von weniger als 50 pg/ml nachweisen. In der Schwangerschaft steigt dann die Konzentration um den Faktor 400.000 auf 210 ng/ml Gesamtestriol an.

### Progesteron

Das Gelbkörperhormon Progesteron induziert die Bildung des Progesteronrezeptors, der aus zwei Isoformen A und B besteht (Kastner et al., 1990). Die Aktivierung der Isoform A bewirkt eine Proliferationshemmung, während die Isoform B eine Proliferationssteigerung ähnlich der durch das Estradiol herbeiführt. Die Daten aus groß angelegten Beobachtungsstudien zeigen, dass das natürliche Progesteron das Brustkrebsrisiko deutlich vermindert (Lambrinoudaki, 2014).

### Testosteron

Glaser et al. (2013) überprüften zwischen 2001 und 2004 im Speichel von Brustkrebspatientinnen (n=357) und Patientinnen mit gutartigen Brusterkrankungen (n=184) die Spiegel von freiem (aktivem) E1, E2, E3, Testosteron, DHEAS, Progesteron und Cortisol.

Die Testosteron- (p<0,001) und DHEAS- (p<0.007) Spiegel waren bei Brustkrebspatientinnen signifikant niedriger als bei den Kontrollen. Die E3-Spiegel (p<0,01) waren in gleicher Weise ebenfalls deutlich niedriger bei Brustkrebspatientinnen.

Im Gegensatz dazu waren die Spiegel für die E1 (p<0,006) und E2 (p<0.005) deutlich erhöht.

Für Progesteron und Cortisol konnte kein Unterschied gefunden werden. Generell haben Frauen in der Postmenopause im Vergleich zu Frauen in der Geschlechtsreife erniedrigte Testosteron- und DHEAS-Spiegel. Dies gilt besonders für postmenopausale Brustkrebspatientinnen. Die E2-Spiegel dagegen waren bei den postmenopausalen Brustkrebspatientinnen deutlich erhöht. Die E1-Spiegel waren sowohl prä- als auch postmenopausal bei den Brustkrebspatientinnen erhöht (Dimitrakakis et al., 2010)

Insgesamt ergibt sich für erhöhte E1- und/oder E2-Spiegel ein gesteigertes Brustkrebsrisiko, während Testosteron und Estriol offensichtlich einen schützenden Effekt haben.

Folgerichtig entwickelte die Gruppe um Glaser et al. (2013) zusammen mit einer Apotheke in Cincinnati, Ohio ein subkutan anwendbares Pellet, in dem einerseits Testosteron allein und andererseits eine Kombination aus Testosteron und Anastrozol, einem Aromatasehemmer, enthalten waren. In diese Dayton-Studie wurden zwischen 2008 und 2010 insgesamt 674 prä- und postmenopausale Patientinnen ohne Brustkrebs eingeschlossen. 257 erhielten Testosteron alleine, während 417 Frauen Testosteron und Anastrozol als Pellet implantiert wurden. Dabei zeigte sich, dass sowohl Testosteron allein als auch die Kombination aus Testosteron und Anastrozol das Brustkrebsrisiko bei prä- und postmenopausalen Frauen senken konnte (Glaser et al., 2013). Estriol und Progesteron wurden bei diesem Behandlungsansatz nicht berücksichtigt.

Dieser präventive Wert geht allerdings einher mit Nebenwirkungen. Ursache hierfür ist die deutliche Verschiebung des Östrogen/Testosteron-Quotienten hin zum Testosteron. Typische Nebenwirkungen, wie auch Glaser et al. berichten, sind eine Vermehrung der Gesichtsbehaarung und das Auftreten von Akne. Nicht berichtet aber vielfach beschrieben werden eine gesteigerte Reizbarkeit, eine Zunahme der Libido und Schlaflosigkeit. Darüber hinaus führen Aromatasehemmer nahezu regelmäßig zu Hitzewallungen, Gelenkbeschwerden, Knochenschmerzen und allgemeine Abgeschlagenheit. Insgesamt ist das Nebenwirkungsprofil bei längerer Anwendung, gerade in der genannten Kombination nicht unerheblich.

Inwieweit eine Tumorfreiheit mit der Kombination von Aromatasehemmer und Testosteron auf längere Sicht erreicht werden kann, ist zum derzeitigen Zeitpunkt offen. Hinweise für eine längerfristige Tumorunterdrückung liefern allenfalls die Japanerinnen und Japaner, die den langanhaltenden Effekt mit den estriolverwandten Phytoestrogenen (Genistein) erreichen. Aus dieser Beobachtung leitet sich die Forderung ab, bei einem therapeutischen oder präventiven Vorgehen in jedem Fall das Estriol einzusetzen. Dies hat zudem den Vorteil, dass testosteronbedingte Nebenwirkungen antagonisiert werden können. In gleicher Weise gilt dies auch für das Progesteron.

### Ziel und Aufgabe der Erfindung

Das Ziel der vorliegenden Erfindung ist es, ein neues Mittel zur Prävention und Therapie hormonsensibler Tumore, insbesondere von Mammakarzinomen oder Prostatakarzinomen zu finden und zur Anwendung bereitzustellen, welches durch seine proliferationshemmende Wirkung die Ausbildung bzw. die Ausbreitung eines Tumors effektiv eindämmt bzw. unterdrückt und gleichzeitig die Lebensqualität der behandelten Patienten deutlich verbessert.

Der Erfindung lag deshalb die Aufgabe zugrunde, ein individuelles wirksames Mittel bereitzustellen, das ein Tumorwachstum signifikant reduziert und dessen Nebenwirkungen deutlich abgemildert sind, so dass es beispielsweise präventiv bedenkenlos während der Postmenopause aber auch in der Geschlechtsreife zusammen mit einem GnRH-Analogon bei Frauen eingesetzt werden kann.

Die Aufgabe der Erfindung wird gemäß Anspruch 1 gelöst. Weitere mögliche Ausführungsformen ergeben sich aus den Unteransprüchen, der Beschreibung, den Zeichnungen und den Beispielen.

### Wesen der Erfindung

Initial galt es die starken Nebenwirkungen, wie Knochen und Muskelschmerzen, unter einer Aromatasehemmer-Therapie zu mildern. Zunächst wurde, belegt durch grundlagenwissenschaftliche Erkenntnisse, die keine tumoranregende Wirkung erwarten ließen, lediglich das Estriol (E3) eingesetzt. Bestärkt durch weitere grundlagenwissenschaftliche Erkenntnisse, wurde wegen der libidosteigernden Wirkung zusätzlich Testosteron verabreicht. Schließlich wurde das Mittel noch durch das nachweislich tumorhemmende Progesteron ergänzt.

Überraschenderweise konnte damit nicht nur eine deutliche Verbesserung der Lebensqualität erzielt werden, vielmehr konnte auch ein deutlicher Rückgang der Tumormanifestationen beobachtet werden.

Dementsprechend basiert die Erfindung auf den eigenen Erkenntnissen, dass sich mit dem im Hauptanspruch 1 genannten Mittel hormonrezeptorexprimierende Tumore ohne weitere Medikation zurückbilden. Belegt wird dies durch die Krankengeschichten in den Ausführungsbeispielen 1-3.

Erfindungsgemäß wird ein Mittel zur Therapie und zur Prävention von Tumorerkrankungen, insbesondere von Mammakarzinomen oder Prostatakarzinomen beansprucht, welches dadurch gekennzeichnet, dass es die Steroidhormone Estriol, Progesteron und Testosteron und einen Aromatasehemmer gemäß Anspruch 1 umfasst.

Es konnte gezeigt werden, dass der Entzug proliferationssteigernder Hormone durch die Verabreichung eines Aromatasehemmers bei gleichzeitiger Gabe der proliferationshemmenden Steroide Estriol (E3), Progesteron und Testosteron überraschenderweise zu einer nachhaltigen Eindämmung des hormonabhängigen Tumorwachstums führt. Die Östrogene gelten generell als proliferationssteigernd, also krebsfördernd. Deshalb war es nicht zu erwarten, dass der Bestandteil Estriol (E3), in der Kombination mit den proliferationshemmenden Steroiden Progesteron und Testosteron eine hemmende Wirkung aufweisen würde.

Darüber hinaus zeigt eine solche Kombination auch einen deutlichen präventiven Effekt.

Als Aromatasehemmer können Letrozol, Exemestan oder Anastrozol gemäß Anspruch 1 verabreicht werden. Als besonders vorteilhaft hat sich die Verwendung von Letrozol herausgestellt.

Die Erfindung konzentriert sich auf die Wirkung der Steroidhormone bei hormonsensiblen Tumorgeweben. Verabreicht wird eine Dreierkombination aus drei proliferationshemmenden Steroiden, namentlich dem schwangerschaftstypischen Estriol E3, dem natürlichen Progesteron und dem Testosteron (perkutan) bei gleichzeitiger Gabe eines Aromatasehemmers gemäß Anspruch 1, z. B.

Letrozol. Hierbei wird die Bildung des proliferationsfördernden Estradiols und des Estrons aus den androgenen Vorstufen verhindert.

Durch die erfindungsgemäße Verabreichung einer Dreierkombination der Steroidhormone Estriol (E3), Progesteron und Testosteron bei gleichzeitiger Gabe eines Aromatasehemmers gemäß Anspruch 1 konnte die Proliferationshemmung deutlich verstärkt werden. Gleichzeitig führt die Verabreichung des erfindungsgemäßen Mittels zu einer ebenso deutlichen Verbesserung der Lebensqualität. Die unerwünschten Nebenwirkungen des Testosterons (z. B. Haarwuchs, Libidosteigerung, Reizbarkeit) werden durch die anderen Komponenten also erheblich abgemildert.

Die Anwendung der Erfindung besteht aus einem kombinierten Vorgehen:
Zu Beginn erfolgt der Nachweis der Expression der Hormonrezeptoren ERα, ERβ, PR A+B, TR im Tumorgewebe.

Entsprechend der Expression der Rezeptoren, erfolgt erfindungsgemäß die Gabe der individuellen Hormonkombination.

Die Mengenverhältnisse der Hormone Estriol, Progesteron und Testosteron in der Kombination können in einem weiten Bereich variieren. Sie können zu gleichen Teilen (1:1:1) enthalten sein. Effektiver ist jedoch Progesteron als Hauptbestandteil in der Kombination zu haben und Testosteron in geringerem Anteil, etwa im Verhältnis 3:1. Estriol ist dazu in deutlich geringerer Menge, etwa einem Zehntel, wirksam, so dass eine Verhältnis von 1 : 20-40 : 5-15, vorzugsweise 1 : 30 : 10, ein bevorzugtes Mittel ist.

Es soll an dieser Stelle betont werden, dass das Ziel der Anwendung eine individuelle Hormonkombination ist, die sich aus der Expression der Rezeptoren und aus anderen vor der Therapie erhobenen Parametern ergibt. Maßgeblich hierbei sind die körperlichen und psychischen Befindlichkeiten, wie Libidosteigerung, Reizbarkeit, Blutdrucksteigerung etc, die eine individuelle Anpassung erforderlich machen.

Die Erfindung betrifft darüber hinaus die Verwendung des erfindungsgemäßen Mittels zur Herstellung eines Arzneimittels für die Prävention und Therapie von malignen Tumoren insbesondere von Mammakarzinomen oder Prostatakarzinomen und/oder für die Therapie von Metastasen.

Die Verwendung des erfindungsgemäßen Mittels ist dadurch gekennzeichnet, dass das Mittel gemäß Anspruch 1 verabreicht wird.

Die Verwendung ist dadurch gekennzeichnet, dass die Steroidhormone Estriol, Progesteron und Testosteron morgens und abends als Salbenformulierung zum Auftragen auf die Haut verabreicht werden. Bevorzugt werden jeweils 2g/100kg Körpergewicht Salbenformulierung der Steroidhormone Estriol, Progesteron und Testosteron morgens und abends in die Haut einmassiert. Als vorteilhaft hat sich herausgestellt, wenn die Steroidhormone Estriol, Progesteron und Testosteron getrennt von dem Aromatasehemmer verabreicht werden. Dabei wird der Aromatasehemmer oral verabreicht.

Nachfolgend wird die vorliegende Erfindung an einem konkreten Anwendungsbeispiel ausführlich dargestellt. Beschrieben wird die Gabe als Hautcreme mit einer Rezeptur von:
Rp.: Estriol 0,1g plus Progesteron 3,0g plus Testosteron 1,0g plus
Salbengrundlage ad 100g
plus 2,5 mg/d Letrozol oral

Morgens und abends wird jeweils 1g dieser Creme in die Haut des Unterarmes einmassiert. Zusätzlich wird jeden Tag ein Aromatasehemmer (z.B. Letrozol 2,5 mg) verabreicht. Diese Kombination mit dem Aromatasehemmer ist bei der Anwendung bei gleichzeitig bestehendem estrogenrezeptorexpressiven Tumor zwingend, um die sofortige Umwandlung des zugeführten Testosterons in die nicht gewünschten Metaboliten Estradiol und Estron zu vermeiden.

Tabelle 1 zeigt bevorzugte Rezepturen von Estriol/Progesteron/Testosteron zusammen mit der täglich zu verabreichenden Dosis im Vergleich zu den handelsüblichen Präparaten Intrinsa, Tostran und Ovestincreme.

**Tabelle 1**

| | **Rezeptur Nr 1: (2g Creme /d)** | **Rezeptur Nr 2: (1 ml Hub /d)** | **Intrinsa/d** | **Tostran 1Hub (0,5 g /d)** | **Ovestincreme (0,5g/d)** |
|---|---|---|---|---|---|
| Estriol | 2 mg) | 15 mg | | | 0,5 mg |
| Progesteron | 60 mg | 45 mg | | | |
| Testosteron | 20 mg | 1,5 mg | 0,3 mg | 10 mg | |

Für die Prävention wird in der Postmenopause bei Frauen die Kombination aus Estriol, Progesteron und Testosteron mit einem Aromatasehemmer gegeben.

Vorteile des erfindungsgemäßen Mittels zur Prävention und Therapie von Tumorerkrankungen durch die Dreiermedikation aus Estriol, Progesteron und Testosteron plus Aromatasehemmer gemäß Anspruch 1 sind:
- deutliche Hemmung der Proliferationsrate, die über einen synergistischen Effekt hinausgeht und damit einhergehend eine Erhöhung der Überlebenschancen
- Abmilderung der Nebenwirkungen von Testosteron und damit einhergehend deutliche Verbesserung der Lebensqualität
- Vermeidung unnötiger Steroidhormongaben
- Deutliche Minderung des Fatigue-Syndroms
- Steigerung der allgemeinen Vitalität
- Deutliche Verminderung metastasenbedingter Schmerzen
- Rückbildung von Tumormanifestationen.

Nachfolgend wird die vorliegende Erfindung spezieller durch Beispiele beschrieben. Die folgenden Beispiele sind jedoch nur zur Erläuterung angegeben und demzufolge ist die vorliegende Erfindung nicht durch sie oder auf sie beschränkt.

### Ausführungsbeispiele

### Beispiel 1

### Diagnose:

C50.2GL/Mammakarzinom des oberen inneren Quadranten
E05.0Z/Hyperthyreose mit diffuser Struma
F41.2G/ängstliche und depressive Verstimmung
E55.9G/Vit D Mangel
R78.8G/Nachweis eines abnormen Lithium-Blutwertes

### Histologie:

lobuläres Mammakarzinom G2
ER 9/12, PR 9/12, HER 2+, FISH nicht amplifiziert, Ki-67 10%.

### Vorgehen:

1. Letrozol (neoadjuvant)
2. Hormoncreme (neoadjuvant Estriol, Progesteron und Testosteron)
3. Dekristol
4. Lithiumortotat

### Anamnese:

57-jährige Patientin, bei der am 22.08.2013 erstmalig ein Mammakarzinom links oben innen per Stanzbiopsie gesichert wurde. Die Patientin hatte selbst seit einem Jahr dort einen Tumor getastet. Die Patientin wurde zur Planung einer neoadjuvanten Therapie vorgestellt. Von ihr wurden allerdings sowohl eine Operation als auch eine Chemotherapie, als auch eine Bestrahlung strikt abgelehnt. In der weiteren Anamnese Schilddrüsen-OP 1998 wegen Überfunktion. Zwei SS-Abbrüche, Menopause mit 53 Jahren, Menarche mit 13 Jahren. Vom 17.-30. LJ orale Ovulationshemmer, mehrere Knieoperationen. Seit 3 Jahren zunehmende Belastungsdyspnoe (COPD).

### Familienanamnese:

unauffällig

### Verlauf:

Mit der Patientin wurde o.g. hormonelle Therapie besprochen. Sie wurde auf den experimentellen Charakter der Behandlung und mögliche Nebenwirkungen ausführlich hingewiesen. Sie gab dazu ihr Einverständnis. Am 04.10.2013 begannen wir o.g. Behandlung. Der Behandlungsverlauf wurde sowohl mit Mammografie als auch Ultraschall verfolgt. Am 04.10.2013 ließ sich an der Brust links oben innen zwischen 12.00 und 13.00 Uhr ein im Durchmesser etwa 5 cm großer derber höckeriger Tumor tasten. Es zeigte sich dort eine diskrete Hauteinziehung. Das Plateauphänomen war positiv. Eine peau d'orange war nicht sichtbar. Im Verlauf verminderte der Tumor unter der Therapie zunehmend seine Größe. Am 18.03.2014 war oben innen lediglich ein Tumor von 1-2 cm Größe tastbar. Die Kontrollmammografie am 27.01.2014 zeigte im Vergleich zum 22.08.2013 einen Rückgang des Tumors von 4,0 auf 2,5 cm. Im Ultraschall der Mammae vom 18.03.2014 zeigte sich im Vergleich zur Untersuchung am 27.09.2013 ein Rückgang von 4,3 auf 1,2 cm (Abbildung 1). Der diskret erhöhte Tumormarker CA 15-3 sank im Verlauf von 32 U/ml (26.09.2013) auf 30 U/ml (16.01.2013). Mit der Patientin wurde erneut die BET besprochen. Sie wollte sich aber weiterhin konservativ behandeln lassen. Ein Anhalt für Fernmetastasen bestand zu diesem Zeitpunkt nicht. Die Lebensqualität der Patientin war ausgezeichnet. Einen ausgeprägten Vitamin D- und Lithiummangel substituierten wir entsprechend.

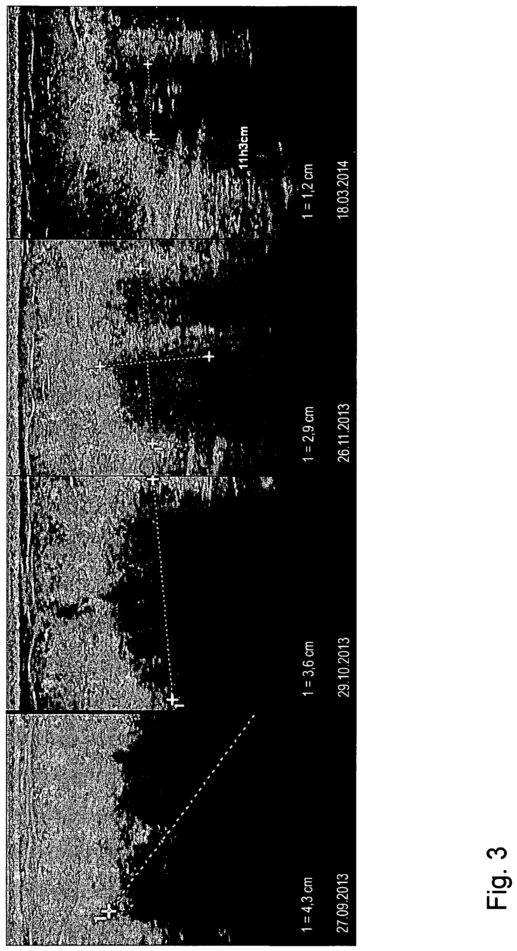

Figur 3 zeigt die Rückbildung eines hormonrezeptorexprimierenden Mammakarzinoms unter Estriol, Progesteron, Testosteron und Aromatasehemmer Letrozol innerhalb von 6 Monaten.

Die weiteren Beispiele zeigen, dass ebenso zusätzlich Metastasen rückgebildet werden können und die begleitenden Schmerzen aufgehoben werden.

### Beispiel 2 - nicht erfindungsgemäß

### Diagnose:

Ovarial-Carcinom
Knochenmetastasen LWK
Deckplattenkompressionsfraktur BWK 12 und LWK 2

### Histologie:

schlecht differenziertes vom Ovar ausgehendes Adenokarzinom
pT3c, pN1 (25/34) MX L1, V1 G3 FIGO IIIC
ER pos.

### Vorgehen:

CarboplatinTaxol - Chemotherapie (15.04.2010-31.01.2013)
Avastin q3 (24.10.2012-31.01.2013)
Xgeva (11.03.2013-04.06.2013)
Bondronat q4 18.06.2013 - andauernd)
Hormoncreme (Estriol, Progesteron, Testosteron, Beginn 04.06.2013 - andauernd) Letrozol (Beginn 25.02.2014 - andauernd)
Dekristol 10.000 i.E./d

### Anamnese:

Die 67-jährige Patientin bemerkte seit Dezember 2009 eine Zunahme des Leibesumfangs. In der Kernspintomografie am 26.01.2010 wurde der Verdacht auf ein Ovarialkarzinom geäußert. Am 9.2.2010 wurde eine Explorativlaparosokopie durchgeführt. Am 26.02.2010 schloss sich die Laparatomie vom Längsschnitt an. Entfernt wurden Gebärmutter und Eierstöcke, großes Netz und retroperitoneale LK. in der weiteren Anamnese
Cholezystektomie, Appendektomie, 1 Spontanpartus, Menopause mit 50 Jahren

### Familienanamnese:

unauffällig

### Verlauf:

Die Patientin absolvierte zunächst die leitliniengerechte Chemotherapie, die relativ gut vertragen wurde. Diese wurde im weiteren Verlauf durch Avastin q3 ergänzt. Im März 2013 traten starke bis stärkste Schmerzen im Bereich der unteren Wirbelsäule auf. Im MRT vom 06.03.2013 zeigten sich alte Deckplattenimpressionen in LWK 2 und 4. im lumbalen und sacralen Bereich. Es ergab sich der Verdacht auf Knochenmetastasen. Wegen zunehmender Schmerzen im Bereich der unteren Wirbelsäule wurde zunächst die Medikation mit Xgeva im Juni 2013 durch Bondronatinfusionen ersetzt.
Am 04.06.2013 wurde mit o.g. Hormoncreme begonnen. Darunter verminderten sich die Schmerzen, sistierten aber nicht gänzlich.
Im MRT vom 11.03.2014 zeigten sich die bekannten alten Wirbelkörperfrakturen. Veränderungen, die auf eine Knochenmetastasierung hinwiesen, waren nicht mehr nachweisbar. Der initial mit 82 U/ml (15.10.2012) deutlich erhöhte Tumormarker kehrte mit 24 U/ml zur Norm (13.08.2013) zurück.
Am 25.02.2014 wurde zusätzlich zur Hormoncreme als "off-label"-Medikation Letrozol gegeben. Darunter verschwanden die Schmerzen komplett. Der Patientin geht es jetzt knapp 4 Jahre nach Erstdiagnose eines ausgedehnten, prognostisch sehr ungünstigen Ovarialkarzinoms unter der Therapie mit der Hormoncreme und dem Letrozol klinisch ausgezeichnet.

### Beispiel 3

### Diagnose:

Mammakarzinom rechts oben außen
Knochenmetastasen
Vit B Mangel

### Histologie:

duktales Mammakarzinom
pT1 (1,3cm), pN1 (1/9), M1 Knochenmetastasen, G2
ER 12/12, PR 9/12, HER2 negativ

### Vorgehen:

1. Letrozol
2. Hormoncreme (Estriol, Progesteron, Testosteron)
3. Bondronat

### Anamnese:

71-jährige Patientin, bei der am 25.08.2005 erstmalig ein Mammakarzinom rechts oben außen nachgewiesen wurde. Am gleichen Tag BET und axilläre Dissektion und Radiatio der Restbrust
Zunächst keine antihormonelle Therapie wegen ausgeprägter Nebenwirkungen im Magen-Darm-Bereich. Wegen erhöhtem Tumormarker (ca. 15-3 93 U/ml) am 25.09.2012 Durchführung eines Knochenszintigramms mit Nachweis multipler Knochenmetastasen an der Schädelkalotte in der BWS, LWS und den Sakralwirbeln. Sonst kein weiterer Metastasennachweis.
2012 Polypenentfernung aus der Harnblase,
seit 2001 Borreliose bekannt
2009 Herpes Zoster
Bei empfindlichem Magen, 2009 Magen- und Darmspiegelung: unauff. drei Schwangerschaften, 1 Abort, Menarch mit 17 Jahren, Menopause mit 50 Jahren

### Familienanamnese:

Tante mütterlicherseits Zervixkarzinom

### Verlauf:

Die Patientin lehnte alle leitlinienbasierten Behandlungen ab.
Im Mai 2013 wurde wegen der Knochenmetastasen eine Behandlung mit Exemestan und Hormoncreme (Estriol, Progesteron und Testosteron und Bondronat) begonnen. Die Schmerzen im Bereich des Knochens sistierten auch ohne Schmerzmittel. Das MRT am 21.02.2014 zeigte eine deutlich rückläufige Knochenmetastasierung. Der Tumormarker CA 15-3 sank von 560 U/ml (11.06.13) auf 485 U/ml (30.01.14). Der Patientin geht es klinisch derzeit sehr gut.

### Beschreibung der Figuren

- **Figur 1**: Schematische Darstellung des Steroidstoffwechsels
- **Figur 2**: Häufigkeit hormonabhängiger Karzinomerkrankungen pro 100.000 und Jahr in Nord- und Südeuropa sowie Ostasien, wo eine Soja-reiche Ernährung vorherrscht (nach IACR/WHO)
- **Figur 3**: Rückbildung eines hormonrezeptorexprimierenden Mammakarzinoms innerhalb von knapp sechs Monaten unter Estriol, Progesteron, Testosteron und Aromatasehemmer Letrozol.

### Literaturverzeichnis

- Howell, A; Cuzick, J; Baum, M; Buzdar, A; Dowsett, M; Forbes, JF; Hoctin-Boes, G; Houghton, J et al.: Results of the ATAC (Arimidex, Tamoxifen, Alone or in Combination) trial after completion of 5 years' adjuvant treatment for breast cancer. The Lancet 2005; 365: 60
- Stettner M, Kaulfuss S, Burfeind P: The relevance of estrogen receptor-beta expression to the antiproliferative effects observed with histone deacetylase inhibitors and phytoestrogens in prostate cancer treatment. Mol Cancer Ther. 2007; 5: 2626
- Zhu BT1, Han GZ, Shim JY, Wen Y, Jiang XR.: Quantitative structure-activity relationship of various endogenous estrogen metabolites for human estrogen receptor alpha and beta subtypes: Insights into the structural determinants favoring a differential subtype binding. Endocrinology. 2006;147:4132
- Wu AH, Wan P, Hankin J, Tseng CC, Yu MC, Pike MC: Adolescent and adult soy intake and risk of breast cancer in Asian-Americans. Carcinogenesis. 2002; 23:1491
- Kastner P, Krust A, Turcotte B, Stropp U, Tora L, Gronemeyer H, Chambon P (1990). "Two distinct estrogen-regulated promoters generate transcripts encoding the two functionally different human progesterone receptor forms A and B". EMBO J. 1990; 9: 1603
- Lambrinoudaki I: Progestogens in postmenopausal hormone therapy and the risk of breast cancer. Maturitas. 2014
- Dimitrakakis C, Zava D, Marinopoulos S, Tsigginou A, Antsaklis A, Glaser R.: Low salivary testosterone levels in patients with breast cancer. BMC Cancer. 2010; 10:547
- Glaser RL, Dimitrakakis C. Reduced breast cancer incidence in women treated with subcutaneous testosterone, or testosterone with anastrozole: a prospective, observational study. Maturitas. 2013; 76:342

## Patentansprüche

1. Mittel zur Verwendung in der Therapie und zur Prävention von Tumorerkrankungen, ausgewählt aus Mammakarzinomen oder Prostatakarzinomen, **dadurch gekennzeichnet, dass** es zum einen eine Salbenformulierung enthaltend die drei Steroidhormone Estriol, Progesteron und Testosteron beinhaltet und zum anderen einen oral zu verabreichenden Aromatasehemmer umfasst.

2. Mittel nach Anspruch 1 zur Verwendung in der Therapie, **dadurch gekennzeichnet, dass** das Mengenverhältnis der Steroidhormone Estriol (E3)/Progesteron/Testosteron 1 : 20-40 : 5-15 beträgt.

3. Mittel nach Anspruch 1 und 2 zur Verwendung in der Therapie, **dadurch gekennzeichnet, dass** das Mengenverhältnis der Steroidhormone Estriol (E3)/Progesteron/Testosteron 1 : 30 : 10 beträgt.

4. Mittel nach Anspruch 1 - 3 zur Verwendung in der Therapie, **dadurch gekennzeichnet, dass** die Konzentration von Estriol (E3) 0,1g/100g, die Konzentration von Progesteron 3g/100g und die Konzentration von Testosteron 1g/1 00g beträgt.

5. Mittel nach Anspruch 1 - 4 zur Verwendung in der Therapie, **dadurch gekennzeichnet, dass** es als oral zu verabreichenden Aromatasehemmer Letrozol, Exemestan oder Anastrozol enthält.

6. Mittel nach Anspruch 5 zur Verwendung in der Therapie, **dadurch gekennzeichnet, dass** es als oral zu verabreichenden Aromatasehemmer Letrozol enthält.

7. Mittel nach Anspruch 6 zur Verwendung in der Therapie, **dadurch gekennzeichnet, dass** die angewendete Menge des Aromatasehemmers Letrozol 2,5 mg/d beträgt.

8. Mittel gemäß Anspruch 1 - 7, **dadurch gekennzeichnet, dass** die Salbenformulierung der Steroidhormone Estriol, Progesteron und Testosteron morgens und abends als Salbenformulierung verabreicht wird.

9. Mittel gemäß Anspruch 1 - 7, **dadurch gekennzeichnet, dass** jeweils 2g/100kg Körpergewicht Salbenformulierung der Steroidhormone Estriol, Progesteron und Testosteron morgens und abends in die Haut einmassiert wird.

## Claims

1. A composition for use in the treatment and prevention of tumour diseases, selected from mammary carcinoma or prostate carcinoma, **characterized in that** on the one hand, it encompasses an ointment formulation containing the three steroid hormones estriol, progesterone and testosterone, and on the other, it comprises an aromatase inhibitor to be administered orally.

2. The composition according to claim 1 for use in treatment, **characterized in that** the quantity ratio between the steroid hormone estriol (E3) : progesterone : testosterone is 1 : 20-40 : 5-15.

3. The composition according to claim 1 and 2 for use in treatment, **characterized in that** the quantity ratio between the steroid hormone estriol (E3) : progesterone : testosterone is 1 : 30 : 10.

4. The composition according to claim 1 - 3 for use in treatment, **characterized in that** the concentration of progesterone is 3g per 100g, and the concentration of testosterone is 1g per 100g.

5. The composition according to claim 1 - 4 for use in treatment, **characterized in that** it contains Letrozol, Exemestan or Anastrozol as an aromatase inhibitor to be administered orally.

6. The composition according to claim 5 for use in treatment, **characterized in that** it contains Letrozol as an aromatase inhibitor to be administered orally.

7. The composition according to claim 6 for use in treatment, **characterized in that** the quantity used of the aromatase inhibitor Letrozol is 2.5mg per day.

8. The composition according to claim 1 - 7, **characterized in that** the ointment formulation of the steroid hormones estriol, progesterone and testosterone is administered in the morning and evening as an ointment formulation.

9. The composition according to claim 1 - 7, **characterized in that** in each case, 2g per 100kg of body weight of the ointment formulation of the steroid hormones estriol, progesterone and testosterone is massaged into the skin in the morning and evening.

## Revendications

1. Médicament destiné à être utilisé dans le traitement et la prévention d'affections tumorales sélectionnées parmi les carcinomes mammaires ou les carcinomes prostatiques, **caractérisé en ce qu'**il contient, d'une part, une formulation de pommade renfermant les trois hormones stéroïdiennes estriol, progestérone et testostérone et, d'autre part, un inhibiteur d'aromatase à administrer par voie orale.

2. Médicament conformément à la revendication 1, destiné à un usage thérapeutique, **caractérisé en ce que** le rapport quantitatif des hormones stéroïdiennes estriol (E3) / progestérone / testostérone est de 1 : 20-40 : 5-15.

3. Médicament conformément à la revendication 1 et 2, destiné à un usage thérapeutique, **caractérisé en ce que** le rapport quantitatif des hormones stéroïdiennes estriol (E3) / progestérone / testostérone est de 1 : 30 : 10.

4. Médicament conformément à la revendication 1 à 3, destiné à un usage thérapeutique, **caractérisé en ce que** la concentration de progestérone s'élève à 3 g/100 g et la concentration de testostérone à 1 g/100 g.

5. Médicament conformément à la revendication 1 à 4, destiné à un usage thérapeutique, **caractérisé en ce qu'**il contient du létrozole, de l'exémestane ou de l'anastrozole, comme inhibiteurs d'aromatase à administrer par voie orale.

6. Médicament conformément à la revendication 5, destiné à un usage thérapeutique, **caractérisé en ce qu'**il contient du létrozole, comme inhibiteur d'aromatase à administrer par voie orale.

7. Médicament conformément à la revendication 6, destiné à un usage thérapeutique, **caractérisé en ce que** la quantité appliquée de létrozole, comme inhibiteur d'aromatase, est de 2,5 mg/d.

8. Médicament conformément à la revendication 1 à 7, **caractérisé en ce que** la formulation de pommade des hormones stéroïdiennes estriol, progestérone et testostérone est administrée le matin et le soin comme pommade.

9. Médicament conformément à la revendication 1 à 7, **caractérisé en ce que** la formulation de pommade des hormones stéroïdiennes estriol, progestérone et testostérone est appliquée en massant la peau le matin et le soir dans la proportion 2 g/100 kg de poids corporel.
